# EUROPÄISCHE PATENTSCHRIFT

(11) **EP 3 511 026 B1**
(45) Veröffentlichungstag und Bekanntmachung des Hinweises auf die Patenterteilung: **18.03.2020**
(21) Anmeldenummer: 18151451.4
(22) Anmeldetag: 12.01.2018
(51) Int. Cl.: A61L 11/00, A61L 9/14, A61L 9/01

(54) **VERFAHREN ZUR BEKÄMPFUNG VON ÜBLEN GERÜCHEN**
METHOD FOR FIGHTING UNPLEASANT ODOURS
PROCÉDÉ DE LUTTE CONTRE DES ODEURS NAUSÉABONDES

(43) Veröffentlichungstag der Anmeldung: 17.07.2019
(73) Patentinhaber: GreenAirSystems GmbH, 58455 Witten (DE)
(72) Erfinder: Hüggenberg, Udo, 45527 Hattingen (DE); Hüggenberg, Franziska, 45527 Hattingen (DE)
(74) Vertreter: Behrendt, Arne

(56) Entgegenhaltungen:
- WO-A2-02/13776
- FR-A1- 3 019 048
- JP-A- 2016 202 557
- US-A1- 2014 162 932

## Beschreibung

Die Erfindung betrifft ein Verfahren zur Bekämpfung von üblen Gerüchen in der Abluft aus Behältern, Räumen oder Anlagen, in denen stark riechende Substanzen wie Fäkalien, Abfälle oder stark riechende Speisen aufbewahrt oder verarbeitet werden, bei welchem in die Abluft ein Fluid in feinverteilter Form eingebracht wird, welches den Geruch der in der Abluft enthaltenen Stoffe physikalisch und/oder chemisch neutralisiert.

Ein Verfahren dieser Art ist beispielsweise aus der DE 100 38 063 A1 bekannt. Bei diesem bekannten Verfahren enthält das in feinverteilter Form versprühte Fluid, hier Wasser, als Wirkstoff Beimengungen in Form von Tensiden und/oder Tensidgemischen, Enzymen und/oder Enzymgemischen, Wasserstoffperoxid, Lösungen von Oxidationsmitteln, Lösungen von Reduktionsmitteln oder amphotere Substanzen, ohne dass genauer angegeben wird, welche Wirkstoffe hier konkret zum Einsatz kommen.

Nach dem Stand der Technik (vergl. WO02/13776A2; JP2016/202575A; FR 3019 048 A1 und US 2014/162932A1) ist es grundsätzlich auch bekannt, zur Bekämpfung von üblen Gerüchen am menschlichen Körper oder in von Menschen bewohnten Räumen ein Fluid zu verwenden, welches Triethylcitrat, 1,5 Propandiol und Di-Propylenglykol-Methylether enthält. Im Stand der Technik werden diese Substanzen allerdings stets in Verbindung mit anderen Wirkstoffen eingesetzt, wie zum Beispiel Parfümstoffen, um nicht nur negative Gerüche zu bekämpfen sondern auch durch Überdeckung und Maskierung einen positiven Geruch zu erzeugen.

Ein weiteres Verfahren der eingangs genannten Art, allerdings hier mit genauer Angabe der Wirkstoffe im Einzelnen, ist aus dem europäischen Patent EP 2 383 398 B1 bekannt, welches auf die gleichen Erfinder wie die vorliegende Patentanmeldung zurückgeht. Bei diesem nach dem Stand der Technik bekannten Verfahren besteht das versprühte Fluid aus einer wässrigen Lösung, welche an Wirkstoffen 2,5 Vol% bis 10 Vol% Glycerin, 0-2,5 Vol% Glykol und 0-2,5 Vol% Essigsäure- n-Butylester enthält.

Obwohl sich das mit diesem Fluid betriebene Verfahren in der Praxis durchaus bewährt hat, lässt es doch im Hinblick auf seine Wirksamkeit und auf die verhältnismäßig große Mindestmenge an Wirkstoff (Glycerin) in dem Fluid zu wünschen übrig, insbesondere wenn es darum geht, besonders stark riechende üble Gerüche zu beseitigen. Dieser Nachteil hat besonderes Gewicht, wenn extrem große Mengen an Abluft behandelt werden müssen, beispielsweise die Abluft aus Anlagen, die sich in großen Hallen oder sogar im Freien befinden, wie zum Beispiel Anlagen für die Müllsortierung, Anlagen für das Mischen von Asphalt, Biogasanlagen, offene Abwasserführungen oder Klärbecken.

Es ist deshalb Aufgabe der Erfindung, das Verfahren der eingangs genannten Art im Hinblick auf seine Wirksamkeit zu verbessern und zugleich die Menge an benötigten Wirkstoffen erheblich zu verringern.

Zur Lösung dieser Aufgabe schlägt die Erfindung ausgehend vom Verfahren der eingangs genannten Art vor, dass das Fluid eine 0,1 %ige bis 8 %ige wässrige Lösung eines Wirkstoffkonzentrates ist, welches zu 55 - 65 Gew% aus Triethylcitrat, zu 17,5 - 22,5 Gew% aus 1,2 Propandiol und zu 17,5 - 22.5 Gew% aus Di-Propylenglykol-Methylether besteht.

Es hat sich überraschenderweise herausgestellt, dass bei der Verwendung eines solchen Wirkstoffkonzentrates die Menge an Wirkstoff in dem wässrigen Fluid erheblich herabgesetzt werden kann, ohne die geruchsvernichtende Wirkung zu beeinträchtigen, und ohne dass weitere Wirkstoffe notwendig sind. Die wesentlich stärkere Unterdrückung bzw. Absorbierung des Geruches ist zurückzuführen auf das Zusammenwirken zwischen dem extrahierend und katalytisch wirkenden Hauptbestandteil Triethylcitrat, dem nebelbildend und hygroskopisch wirkenden 1,2-Propandiol und dem als Lösungsmittel wirkenden und ebenfalls hygroskopisch wirkenden Di-Propylenglykol-Methylether. Insbesondere die katalytische Wirkung von Triethylcitrat bewirkt, dass die Gerüche nicht nur überdeckt oder maskiert werden, sondern durch chemische Umsetzung der geruchsbildenden Stoffe vernichtet werden. Diese geruchsbildenden Stoffe sind insbesondere polyzyklische aromatische Kohlenwasserstoffe (PAH) mit kondensierten Ringsystemen, die durch die oben angesprochenen Wirkungen von Triethylcitrat aufgebrochen werden und hierdurch zum größten Teil nicht nur ihre Geruchswirkung verlieren, sondern auch durch natürliche Abbauprozesse schnell abbaubar gemacht werden.

Die oben erläuterten positiven Wirkungen der neuen Wirkstoffkombination sind aber nicht nur bei Verwendung des Wirkstoffkonzentrates verdünnt in einer wässrigen Lösung erreichbar, sondern auch erreichbar, wenn das Wirkstoffkonzentrat auf andere Art und Weise ausreichend fein verteilt in die übelriechende Luft eingebracht wird. Insbesondere ist es gut möglich, für eine ausreichende Feinverteilung der Wirkstoffe dadurch zu sorgen, dass das Konzentrat in einer angemessenen Menge mit einem geeigneten Treibgas vermischt wird und mithilfe dieses Treibgases aus einer geeigneten Spraydose versprüht wird. Diese Möglichkeit ist insbesondere geeignet, wenn der üble Geruch von kleinen Luftmengen bekämpft werden soll.

Gegenstand der Erfindung ist also weiterhin ein Verfahren zur Bekämpfung von üblen Gerüchen in der Luft von Räumen, in denen stark riechende Substanzen wie Fäkalien, Abfälle oder stark riechende Speisen aufbewahrt oder verarbeitet werden, bei welchem in die Raumluft ein Fluid in feinverteilter Form eingebracht wird, welches den Geruch der in der Raumluft enthaltenen Stoffe physikalisch und oder chemisch neutralisiert, wobei sich dieses Verfahren dadurch kennzeichnet, dass das Fluid ein Gemisch aus einem Treibgasgemisch und einem Wirkstoffkonzentrat ist, welches zu 0,1 - 8 Gew% in dem Treibgasgemisch enthalten ist, wobei das Treibgasgemisch zu 55 - 80 Gew% aus Butan, zu 15 - 40 Gew% aus Propan und zu 1 - 25 Gew% aus Isobutan besteht , und das Wirkstoffkonzentrat zu 55 - 65 Gew% aus Triethylcitrat, zu 17,5 - 22,5 Gew% aus 1,2 Propandiol und zu 17,5 - 25,5 Gew% aus Di-Propylenglylol-Methylether besteht.

Folgende Versuche zur Bekämpfung von üblen Gerüchen mit den Verfahren gemäß der Erfindung waren außerordentlich erfolgreich:

### Beispiel 1:

In der Halle einer Müllsortieranlage für Leichtverpackungs-Müll (LVP-Sortieranlage) wurde über Ventilatoren und Zweistoffdüsen ein feinverteilter Nebel bestehend aus 2,5 Gew% Triethylcitrat, 1,25 Gew% Propandiol 1,25 Gew% Di-Propylenglykol-Methylether und 95 Gew% Wasser versprüht. Durch diese Maßnahme konnten die üblen Gerüche in bis an die Wahrnehmungsgrenze eliminiert werden. Dabei waren die Bedüsungseinrichtungen gezielt im Bereich der Hallentore angeordnet, die wegen des steten Verkehrs ständig offenbleiben mussten. Durch diese gezielte Anordnung der Geruchsbeseitigungsanordnung im Bereich der Hallentore wurde eine effektive Geruchssperre erreicht, sodass Anrainer durch die üblen Gerüche aus dieser LVP-Sortieranlage nicht mehr gestört wurden.

### Beispiel 2:

Im Bereich einer weitgehend im Freien befindlichen Verladeanlage für Asphalt an einem Asphaltmischwerk wurden im Bereich der Beladeklappen Bedüsungseinrichtungen installiert, mittels derer in die beim Beladevorgang entstehende Abluft ein feiner Nebel bestehend aus 1 Gew% Triethylcitrat, 0,5 Gew% Propandiol, 0,5 Gew% Di-Propylenglykol- Methylether und 98 Gew% Wasser eingesprüht wird. Trotz des hier sehr niedrigen Gehaltes an Wirkstoff in dem Fluid konnte durch diese Maßnahme eine nahezu vollständige Beseitigung des Asphaltsgeruchs erreicht werden.

### Beispiel 3:

In Haushalten mit Heimtierhaltung wurden zur Beseitigung von üblen Gerüchen Spraydosen eingesetzt, aus denen ein Gemisch aus Treibgas und Wirkstoffkonzentrat gemäß der Erfindung in feinverteilter Form in der Luft der geruchsbelasteten Räume versprüht wurde. Auch hier ergab sich eine nahezu vollständige Beseitigung der üblen Gerüche.

## Patentansprüche

1. Verfahren zur Bekämpfung von üblen Gerüchen in der Abluft aus Behältern, Räumen oder Anlagen, in denen stark riechende Substanzen wie Fäkalien, Abfälle oder stark riechende Speisen aufbewahrt oder verarbeitet werden, bei welchem in die Abluft ein Fluid in feinverteilter Form eingebracht wird, welches den Geruch der in der Abluft enthaltenen Stoffe physikalisch und/oder chemisch neutralisiert,
**dadurch gekennzeichnet,**
**dass** das Fluid eine 0,1 %ige bis 8 %ige wässrige Lösung eines Wirkstoffkonzentrates ist, welches zu 55 - 65 Gew% aus Triethylcitrat, zu 17,5 - 22,5 Gew% aus 1,2 Propandiol und zu 17,5 - 22.5 Gew% aus Di-Propylenglykol-Methylether besteht.

2. Verfahren zur Bekämpfung von üblen Gerüchen in der Luft in Räumen, in denen stark riechende Substanzen wie Fäkalien, Abfälle oder stark riechende Speisen aufbewahrt oder verarbeitet werden, bei welchem in die Raumluft ein Fluid in feinverteilter Form eingebracht wird, welches den Geruch der in der Raumluft enthaltenen Stoffe physikalisch und/oder chemisch neutralisiert, **dadurch gekennzeichnet, dass** das Fluid ein Gemisch aus einem Treibgasgemisch und einem Wirkstoffkonzentrat ist, welches zu 0,1 - 8 Gew% in dem Treibgasgemisch enthalten ist, wobei das Treibgasgemisch zu 55 - 80 Gew% aus Butan, zu 15 - 40 Gew% aus Propan und zu 1 - 25 Gew% aus Isobutan besteht, und das Wirkstoffkonzentrat zu 55 - 65 Gew% aus Triethylcitrat, zu 17,5 - 22,5 Gew% aus 1,2 Propandiol und zu 17,5 - 25,5 Gew% aus Di-Propylenglylol-Methylether besteht.

3. Verwendung eines Konzentrates bestehend aus
- 55 - 65 Gew% Triethylcitrat
- 17,5 - 22,5 Gew% 1,2 Propandiol
- 17,5 - 22.5 Gew% Di-Propylenglykol-Methylether
zur Bekämpfung von üblen Gerüchen in der Abluft aus Behältern, Räumen oder Anlagen in denen stark riechende Substanzen wie Fäkalien, Abfälle oder stark riechende Speisen aufbewahrt oder verarbeitet werden.

## Claims

1. Method for controlling foul odours in the outgoing air from containers, rooms or plants in which strongly smelling substances such as faecal matter, wastes or strongly smelling foods are kept or processed, by introducing into the outgoing air a fluid in finely divided form which physically and/or chemically neutralizes the odour of the substances present in the outgoing air,
**characterized**
**in that** the fluid is a 0.1% strength to 8% strength aqueous solution of an active ingredient concentrate which consists to an extent of 55 - 65 wt% of triethyl citrate, 17.5 - 22.5 wt% of 1,2-propanediol and 17.5 - 22.5 wt% of dipropylene glycol methyl ether.

2. Method for controlling foul odours in the air in rooms in which strongly smelling substances such as faecal matter, wastes or strongly smelling foods are kept or processed, by introducing into the room air a fluid in finely divided form which physically and/or chemically neutralizes the odour of the substances present in the room air, **characterized in that** the fluid is a mixture of a propellant gas mixture and an active ingredient concentrate which is present at 0.1 - 8 wt% in the propellant gas mixture, where the propellant gas mixture consists to an extent of 55 - 80 wt% of butane, 15 - 40 wt% of propane and 1 - 25 wt% of isobutane and the active ingredient concentrate consists to an extent of 55 - 65 wt% of triethyl citrate, 17.5 - 22.5 wt% of 1,2-propanediol and 17.5 - 25.5 wt% of dipropylene glycol methyl ether.

3. Use of a concentrate consisting of
- 55 - 65 wt% of triethyl citrate
- 17.5 - 22.5 wt% of 1,2-propanediol
- 17.5 - 22.5 wt% of dipropylene glycol methyl ether for controlling foul odours in the outgoing air from containers, rooms or plants in which strongly smelling substances such as faecal matter, wastes or strongly smelling foods are kept or processed.

## Revendications

1. Procédé pour la lutte contre des mauvaises odeurs dans l'air sortant de récipients, d'espaces ou d'installations, dans lesquels des substances fortement odorantes telles que des matières fécales, des déchets ou des denrées alimentaires fortement odorantes sont conservé(e)s ou sont traité(e)s, dans lequel un fluide sous forme finement divisée est incorporé à l'air sortant, qui neutralise physiquement et/ou chimiquement l'odeur des matières contenues dans l'air sortant, **caractérisé en ce que** le fluide est une solution aqueuse d'une concentration de 0,1 % à 8 % d'un concentré de principe actif, qui est constitué de 55 à 65 % en poids de citrate de triéthyle, de 17,5 à 22,5 % en poids de 1,2-propanediol et de 17,5 à 22,5 % en poids de di-propylèneglycol-méthyléther.

2. Procédé pour la lutte contre des mauvaises odeurs dans l'air dans des espaces, dans lesquels des substances fortement odorantes telles que des matières fécales, des déchets ou des denrées alimentaires fortement odorantes sont conservées ou sont traitées, dans lequel un fluide sous forme finement divisée est incorporé à l'air ambiant, lequel fluide neutralise physiquement et/ou chimiquement l'odeur des matières contenues dans l'air ambiant, **caractérisé en ce que** le fluide est un mélange d'un mélange de gaz propulseur et d'un concentré de principe actif, qui est contenu dans le mélange de gaz propulseur à raison de 0,1 à 8 % en poids, le mélange de gaz propulseur étant constitué de 55 à 80 % en poids de butane, de 15 à 40 % en poids de propane et de 1 à 25 % en poids d'isobutane, et le concentré de principe actif étant constitué de 55 à 65 % en poids de citrate de triéthyle, de 17,5 à 22,5 % en poids de 1,2-propanediol et de 17,5 à 25,5 % en poids de di-propylèneglycol-méthyléther.

3. Utilisation d'un concentré constitué de
- 55 à 65 % en poids de citrate de triéthyle
- 17,5 à 22,5 % en poids de 1,2-propanediol
- 17,5 à 22,5 % en poids de di-propylèneglycol-méthyléther
pour la lutte contre des mauvaises odeurs dans l'air sortant de récipients, d'espaces ou d'installations, dans lesquels des substances fortement odorantes telles que des matières fécales, des déchets ou des denrées alimentaires fortement odorantes sont conservé(e)s ou sont traité(e)s.
